# FASCICULE DE BREVET EUROPEEN

(11) **EP 2 987 472 B1**
(45) Date de publication et mention de la délivrance du brevet: **15.03.2017**
(21) Numéro de dépôt: 15187777.6
(22) Date de dépôt: 15.11.2012
(51) Int. Cl.: A61F 5/01

(54) **ORTHÈSE ACTIVE DU GENOU**
AKTIVE KNIEORTHESE
ACTIVE KNEE ORTHOSIS

(30) Priorité: 23.11.2011 FR 1160709
(43) Date de publication de la demande: 24.02.2016
(62) Demande divisionnaire de: 12795556.5
(73) Titulaire: De Cortanze, André, 83150 Bandol (FR); Lecurieux-Clerville, Roger, 13008 Marseille (FR)
(72) Inventeur: De Cortanze, André, 83150 Bandol (FR); Lecurieux-Clerville, Roger, 13008 Marseille (FR)
(74) Mandataire: Novagraaf Technologies

(56) Documents cités:
- WO-A1-00/44322
- WO-A1-2010/032476
- US-A1- 2003 100 853
- US-B1- 6 540 708

## Description

La présente invention a pour objet une orthèse active du genou comprenant deux parties articulées entre elles et qui, munie chacune d'au moins un moyen d'attache, se fixent l'une respectivement sur une cuisse et l'autre sur une jambe d'un membre inférieur d'une personne ou patient pour, d'une part, renforcer extérieurement le genou, maintenir en place l'articulation et soulager les pathologies osteoarticulaires et, d'autre part, corriger une déviation latérale anormale de la courbure du membre inférieur, tel que de type valgus ou varus, d'où sa qualification d'active.

Le domaine technique est la réalisation et l'utilisation d'une telle orthèse ou appareil orthopédique externe pour le genou et qui peut être en mis en place par la personne elle-même.

On connaît dans ce domaine de nombreux appareils ayant fait l'objet pour la plupart de dépôt de demandes de brevet : on peut citer par exemple la demande FR 2 872 205 de la société AXMED qui décrit une orthèse dont les parties articulées entre elles comportent chacune des montants rigides latéraux réunis entre eux par une embrase rigide et dont l'un au moins des montants est réglable en longueur de sorte que, les montants pouvant avoir ainsi des longueurs différentes, la partie de l'orthèse correspondante présente une déviation latérale apte à corriger un varus de valgus.

La demande de brevet UK 2 136 294 de la société NORTHERN SCIENT EQUIPMENT décrit également une orthèse pour personne souffrant de genoux valgus, et comprenant un dispositif télescopique disposé d'un côté du genou et combiné avec des sangles qui entourent le genou par l'autre côté et forcent alors celui-ci vers le dispositif télescopique pour corriger la déviation anormale du genou.

On connaît également la demande de brevet US2011004135 de M. KAUSEK qui décrit une orthèse comprenant d'un côté une articulation et un poussoir combinés avec des sangles qui entourent le genou et qui tirant ainsi de l'autre côté du poussoir sur le fémur et le tibia, tendent à corriger, comme dans la demande de brevet UK2136294 ci-dessus, la déviation anormale du genou et/ou à soulager au moins l'appui de celui-ci du côté opposé au poussoir.

Le brevet US 6,540,708 B1 divulgue une orthèse de genou permettant une compression ou distraction du genou en utilisant un mouvement hélicoïdal.

La demande de brevet US 2003/0100853 A1 divulgue une orthèse de genou avec un engrenage.

Tous ces appareils et bien d'autres permettent certes de corriger ou soulager des problèmes de genou mais soit ils sont inconfortables, surtout dans la position de flexion, soit leur action n'est que partielle et/ou même ils peuvent provoquer des effets secondaires préjudiciables à l'articulation.

Le problème posé est donc de réaliser un appareil orthopédique ou orthèse de genou qui corrige les déviations latérales anormales de celui-ci, tout en renforçant l'articulation, et soulage la pression d'écrasement interosseux entre les os du genou en position d'extension, et qui soit confortable pour le patient, surtout en position de flexion, et dont l'action de correction et de renfort sur le genou soit la plus complète sans créer d'effets secondaires.

Une solution au problème posé est une orthèse de genou comprenant deux parties, la première dite supérieure et la deuxième dite inférieure, munies chacune au moins d'un moyen d'attache reliant des pièces supports, apte à les fixer respectivement sur une cuisse et sur une jambe d'un membre inférieur d'une personne présentant une déviation latérale initiale anormale, et ayant une forme générale circulaire adaptable respectivement à la dite cuisse et à la dite jambe et avec le même axe respectivement que celles-ci, et tel qu'ainsi elles suivent le mouvement de cette cuisse et de cette jambe ; les deux dites parties sont reliées par des moyens d'articulation bicentrique, destinés à être positionnés latéralement de part et d'autre du genou et adaptés à autoriser le pivotement des parties supérieure et inférieure autour chacune d'un axe commun entre les extrémités des pièces supports de celles-ci et une pièce intermédiaire formant biellette du moyen d'articulation correspondant ; selon l'invention, un premier moyen d'articulation, correspondant au côté de l'orthèse vers lequel s'incline la déviation initiale, d'une part écarte les deux axes communs entre sa biellette et les pièces supports des parties supérieure et inférieure, quand celles-ci sont pivotées dans le sens de tendre à l'alignement de leurs axes, et, d'autre part, rapproche ces deux axes communs, quand lesdites parties supérieure et inférieure sont pivotées dans le sens d'augmenter l'angle relatif entre leurs axes ; et le deuxième moyen d'articulation, correspondant au côté de l'orthèse opposée à la déviation initiale, comporte une pièce poussoir qui s'avance vers le premier moyen d'articulation situé de l'autre côté de l'orthèse, quand les parties supérieure et inférieure sont pivotées dans le sens de tendre à l'alignement de leurs axes, et s'éloigne de ce premier moyen d'articulation quand les dites parties supérieure et inférieure sont pivotées dans le sens d'augmenter l'angle relatif entre leurs axes.

Dans un mode préférentiel de réalisation, ladite orthèse suivant l'invention comporte un câble reliant l'axe commun, entre la biellette du moyen d'articulation, correspondant au côté de l'orthèse opposée à la déviation initiale, et la partie supérieure de l'orthèse, avec l'extrémité distale d'un bras formant une des pièces supports de la partie inférieure de l'orthèse et disposé dans une direction quasi parallèle à l'axe de celle-ci : la longueur du câble est déterminée pour que celui-ci exerce une traction donnée sur les points d'attache de ses deux extrémités quand les parties supérieure et inférieure sont pivotées l'une par rapport à l'autre dans le sens de tendre à l'alignement de leurs axes, et au contraire soit détendu quand les parties supérieure et inférieure sont pivotées en sens inverse.

Le résultat est une nouvelle orthèse active du genou qui répond au problème posé, d'une part en corrigeant les déviations latérales anormales du genou et en soulageant la pression d'écrasement interosseux, qui peut aller jusqu'au contact des os de celui-ci, en position d'extension de l'articulation, et, d'autre part, en relâchant les forces mises en oeuvre pour ces actions de correction et de soulagement de pression en position de flexion de l'articulation, ce qui procure au patient portant l'orthèse un confort d'utilisation.

De plus, la combinaison des trois différents dispositifs décrits ci-dessus, et précisés dans la description ci-après, permet une action d'ensemble de correction, de renfort et de soulagement de pression plus complète et efficace que dans toutes les orthèses connues à ce jour : et comme indiqué précédemment, ces trois dispositifs en combinaison ne jouent leur rôle en position d'extension du genou qu'entre 0 et 35° environ et relâchent leurs efforts de traction et de poussée au-delà, permettant au genou de reprendre une position confortable pour le patient dans sa position de flexion.

Les avantages évoqués ci-dessus prouvent ainsi l'intérêt de cette nouvelle orthèse ou genouillère dont la description et les figures ci-jointes en donnent un exemple de réalisation.

D'autres modes de réalisations sont cependant possibles dans le cadre de la portée de la présente invention.
La figure 1 est une vue de face en coupe d'un genou en position d'extension et dans lequel un côté du condyle fémoral est, par déficience osteoarticulaire, en contact avec le plateau tibial, créant un déséquilibre latéral et une déviation anormale de l'articulation et générant des douleurs.
Les figures 2 sont des vues en coupe du genou de la figure 1 équipé d'une orthèse suivant l'invention, d'une part en position d'extension et de correction grâce à la prothèse, et, d'autre part, en position de flexion.
Les figures 3 sont des vues de côté et en perspective d'un exemple de réalisation d'un premier moyen d'articulation bicentrique latéral de l'orthèse suivant l'invention apte à écarter les parties supérieure et inférieure de celle-ci, en position d'extension du genou.
Les figures 4 sont des vues perspectives d'un exemple de réalisation d'un deuxième moyen d'articulation bicentrique latéral de l'orthèse suivant l'invention apte à pousser contre le genou en position d'extension de celui-ci.
Les figures 5 sont des vues de côté et de profil d'un exemple de réalisation et de mise en oeuvre d'un câble de traction latéral de l'orthèse suivant l'invention en position d'extension et de flexion du genou.
Les figures 6 sont des vues de face d'une orthèse suivant l'invention en représentation plus agrandie et dans les mêmes positions que sur les figures 2.
Les figures 7 sont des vues de côté et de profil d'un autre exemple de réalisation et de mise en oeuvre d'un câble de traction latéral de l'orthèse suivant l'invention en position d'extension du genou.

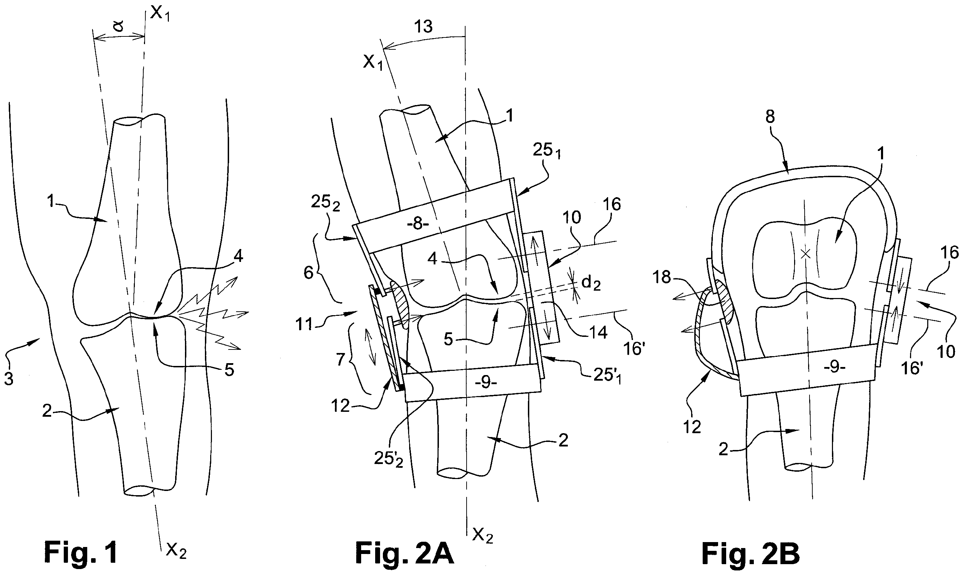

L'objectif de la présente invention est, comme défini précédemment, de corriger la déviation initiale latérale anormalement importante d'angle Y, tel que représenté à titre d'exemple sur la figure 1 et représentant ce que l'on appelle une jambe arquée 3, entre l'axe X₁ de la cuisse ou du fémur 1 et celui de X₂ de la jambe ou du tibia 2, en position d'extension du genou sans orthèse, et soulager la pression, ici de contact, entre les os du genou, soit entre le condyle fémoral 4 et le plateau tibial 5.

Sur la figure 2A le même genou équipé de l'orthèse suivant l'invention est représenté redressé par celle-ci de sa déviation anormale latérale Y, après rotation 13, certes représenté d'une manière exagérée sur cette figure, pour plus de compréhension ; en position de flexion, comme représenté sur la figure 2B, le relâchement de l'ensemble des actions de l'orthèse sur l'articulation apporte le confort souhaité au patient.

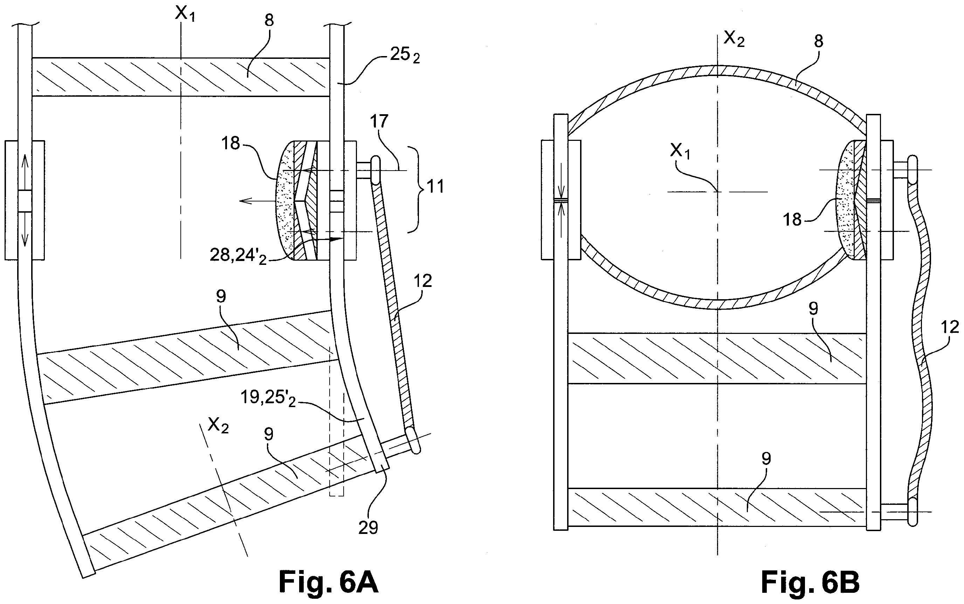

Pour cela, l'orthèse du genou comprend deux parties 6, 7, la première 6 dite supérieure et la deuxième 7 dite inférieure, munies chacune d'au moins un moyen d'attache tel que des embases 8, 9 reliant des pièces supports 25, 25' et apte à les fixer respectivement sur la cuisse et sur la jambe d'un membre inférieur de la personne ou patient qui présente une déviation latérale anormale Y, comme sur la figure 1 ; ces moyens d'attache ont une forme générale circulaire adaptable respectivement à ladite cuisse et ladite jambe et avec le même axe respectivement X₁ et X₂ que celle-ci ; tel que schématisé sur la figure 6B, cette forme générale circulaire est représentée par la vue axiale du moyen d'attache, qui peut être une sangle 8 sur et entourant la cuisse, qui, elle, est non représentée sur cette figure, et qui est en position de flexion par rapport à la jambe sur laquelle est fixée la partie inférieure de la prothèse par les deux moyens d'attache 9.

Ainsi, ces parties supérieure 6 et inférieure 7 de l'orthèse suivent le mouvement de cette cuisse et de cette jambe et les deux dites parties 6, 7 sont reliées par des moyens d'articulation 10, 11 bicentriques, destinés à être positionnés latéralement de part et d'autre du genou et adaptés à autoriser le pivotement des parties supérieure 6 et inférieure 7 autour chacune d'un axe commun 16, 16' pour un premier moyen 10 d'articulation, et 17, 17' pour le deuxième moyen 11 d'articulation, entre les extrémités 24, 24' des supports 25, 25' de celles-ci et une pièce intermédiaire formant biellette 14, 15 du moyen d'articulation correspondant 10, 11.

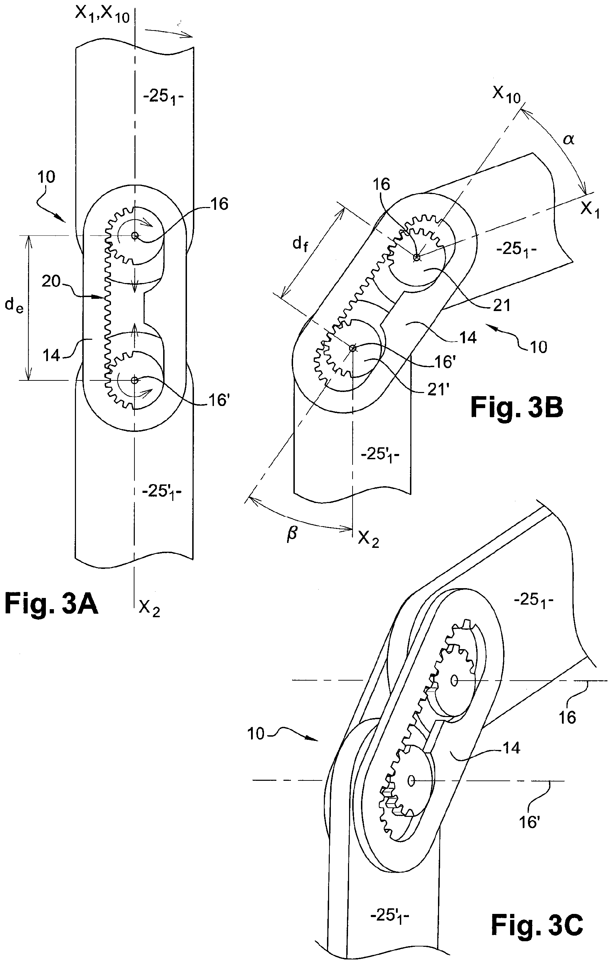

Le premier moyen d'articulation 10, correspondant au côté de l'orthèse vers lequel s'incline la déviation initiale γ de la figure 1, soit du côté que l'on peut qualifier de concave du genou et qui est celui du pincement de l'articulation, où le plateau tibial 5 est trop proche et même jusqu'à être en contact avec le condyle 4 fémoral. Ce premier moyen d'articulation 10, tel que représenté sur les figures 2 et 3, d'une part, écarte les deux axes communs 16, 16' entre sa biellette 14 et les pièces supports 25₁, 25'₁ des parties supérieure 6 et inférieure 7, quand celles-ci sont pivotées dans le sens de tendre à l'alignement de leurs axes X₁ et X₂ et, d'autre part, rapproche ces deux axes communs quand lesdites parties supérieure et inférieure sont pivotées dans le sens d'augmenter l'angle α + β relatif entre leurs axes X₁ et X₂: l'angle α est à considérer entre l'axe X₁ de la pièce support 25₁ et l'axe X₁₀ de la biellette 14, et l'angle β entre l'axe X₂ de la pièce support 25'₁ et l'axe X₁₀ de cette biellette 14.

Suivant le mode de réalisation de ces figures 3, la biellette 14 comporte un logement intérieur doté au moins d'une crémaillère 20 et dans lequel sont guidées et tournent deux roues dentées 21 coopérant avec cette crémaillère, lesquelles roues dentées étant fixées et solidaires chacune de l'extrémité 24₁ et 24'₁ d'une pièce support 25₁, 25'₁ respectivement des parties supérieure 6 et inférieure 7 de l'orthèse.

Ainsi, tel que représenté sur la figure 3A en position d'extension du genou, les pièces supports 25₁ et 25'₁ sont écartées au maximum l'une de l'autre par un écartement maximum « de » des axes 16 et 16', alors qu'en position de flexion ou semi-flexion, comme sur la figure 3B, cette distance entre les axes 16 et 16' diminue « d_{f} » par la rotation des roues dentées 21 le long de la crémaillère 20, rapprochant ainsi les pièces supports 25₁ et 25'₁, et soulageant ainsi l'articulation.

Ainsi, à titre d'exemple, la différence d'écartement entre les axes 16 et 16' (dₑ-d_{f}) est de 4 à 8 mm pour obtenir un écartement osseux d₂ (suivant la figure 2A), entre les bords du plateau tibial 5 et du condyle fémoral 4, de 2 à 4 mm compte tenu de la distraction des parties « molles » comme la peau et les muscles.

Dans un autre mode de réalisation, non représenté sur une figure jointe, la biellette 14 peut comporter un logement intérieur doté de deux cavités dans chacune desquelles est guidée et tourne une came fixée et solidaire chacune de l'extrémité 24₁, 24'₁ d'une pièce support respectivement des parties supérieure 6 et inférieure 7 de l'orthèse, chaque came s'appuyant sur la forme intérieure de sa cavité en forme de rampe permet lors de sa rotation d'obtenir les mêmes effets de rapprochement et d'écartement que le mode de réalisation des figures 3.

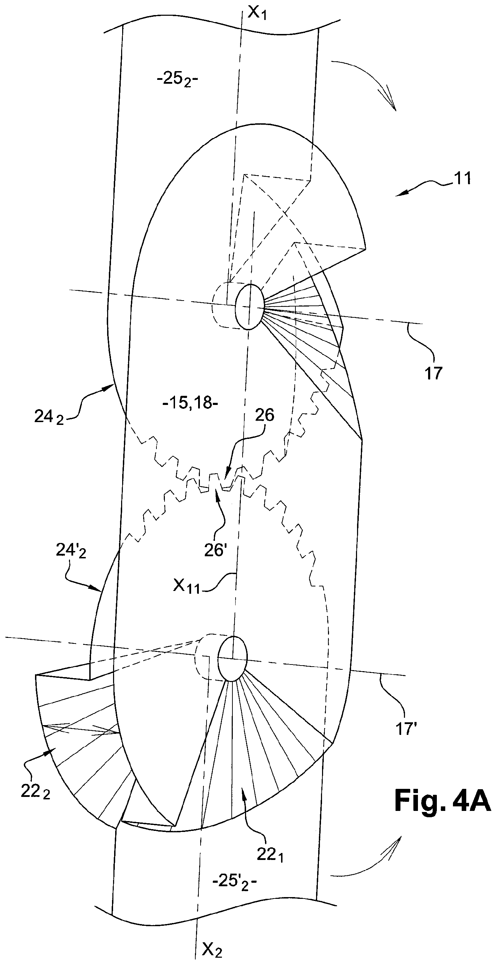

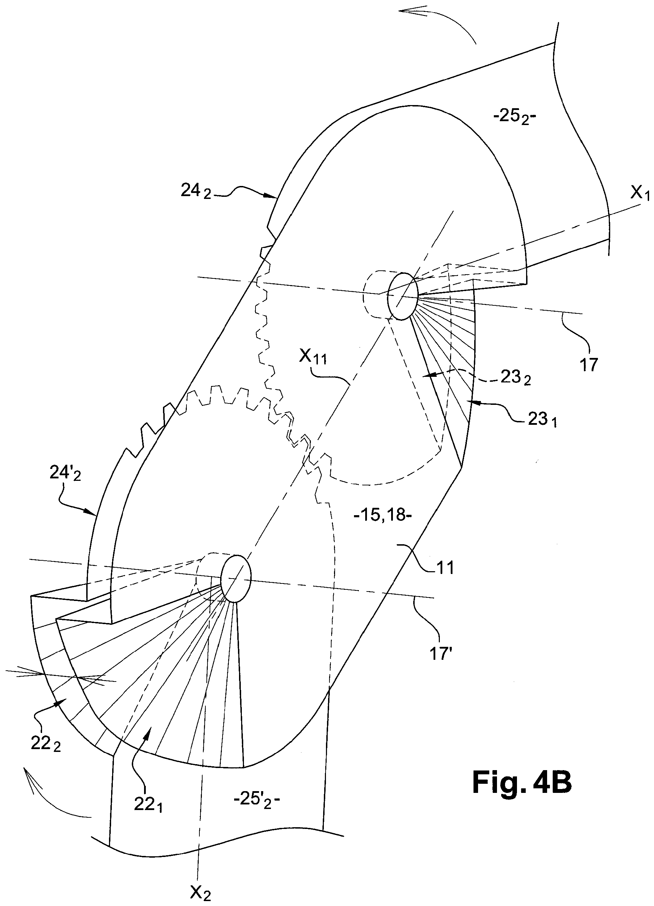

Le deuxième moyen d'articulation 11 qui est positionné du côté de l'orthèse opposé à la déviation initiale γ de la figure 1, soit de l'autre côté du genou par rapport à celui où est disposé le premier moyen d'articulation 10, c'est-à-dire du côté convexe du genou correspondant à l'écartement de l'articulation où le plateau tibial 5 est trop éloigné du condyle 4 fémoral. Ce deuxième moyen d'articulation comporte une pièce poussoir 18 qui s'avance vers le premier moyen d'articulation 10 situé donc de l'autre côté de l'orthèse, quand les parties supérieure 6 et inférieure 7 sont pivotées dans le sens de tendre à l'alignement de leurs axes X₁ et X₂, soit en position d'extension du genou, et s'éloigne de ce premier moyen d'articulation quand lesdites parties supérieure 6 et inférieure 7 sont pivotées dans le sens d'augmenter l'angle α + β relatif entre leurs axes X₁ et X₂, soit en position de flexion du genou : les angles α + β sont ceux déjà définis précédemment, entre l'axe X₁₁ de la biellette 15 de ce deuxième moyen d'articulation 11 et respectivement les axes X₁ et X₂ des parties supérieure 6 et inférieure 7, soit des pièces supports correspondantes 25₂ et 25'₂, tel que représenté sur les figures 4A et 4B.

Les figures 4A et 4B représentent un exemple de réalisation de ce deuxième moyen d'articulation 11 dans lequel la pièce poussoir 18 forme la biellette 15 de l'articulation : la surface de cette biellette 15 est de forme hélicoïdale sur au moins un quart de sa surface 22₁, 23₁ autour de ces deux axes 17, 17', lesquelles formes 22, 23 constituent des rampes qui s'appuient et coopèrent avec des extrémités 24₂, 24'₂ des pièces supports 25₂, 25'₂ fixées respectivement aux parties supérieure et inférieure de l'orthèse et de forme également hélicoïdale 22₂, 23₂ formant également rampe, de même pas et de même axe commun 17, 17' ; lesdites extrémités 24₂, 24'₂ des pièces supports comportant chacune une portion de couronne dentée 26, 26' de même axe 17, 17' et qui coopèrent l'une avec l'autre.

Ainsi, dans la position de flexion du genou, tel que représenté sur la figure 4B, les formes ou rampes hélicoïdales de la biellette 15 sont entièrement imbriquées dans celles des pièces supports 25₂, 25'₂, et la biellette 15 est ainsi dans une position la plus rapprochée des extrémités 24₂ et 24'₂ de ces pièces supports ; alors que sur la représentation des figures 4A en position d'extension de l'articulation du genou, les pièces supports 25₂ et 25'₂ ayant pivoté par rapport à la biellette 15, les formes hélicoïdales, s'appuyant les unes sur les autres en tournant comme si l'on les dévissait l'une par rapport à l'autre, écartent au maximum la biellette 15 des extrémités 24₂, 24'₂ des pièces supports : cet écartement se traduit par un effet de poussée contre le genou, puisque les pièces supports 25₂, 25'₂ sont fixées sur la cuisse et la jambe et ne peuvent s'en écarter, c'est donc la biellette 15 qui joue le rôle de la pièce poussoir 18, tel que représenté sur les figures 2A et 6A.

En plus de la combinaison des deux moyens d'articulation 10, 11, tel que ceux décrits ci-dessus, l'orthèse suivant l'invention comporte un câble 12 reliant l'axe commun 17 entre la biellette 15 du deuxième moyen d'articulation 11 et la pièce support 25₂ de la partie supérieure 6 de l'orthèse, avec un point d'attache 30 fixé sur l'extrémité distale 29 d'un bras 19 formant une des pièces supports 25'₂ de la partie inférieure 7 de l'orthèse, et disposée dans une direction quasi parallèle à l'axe X₂ de celle-ci.

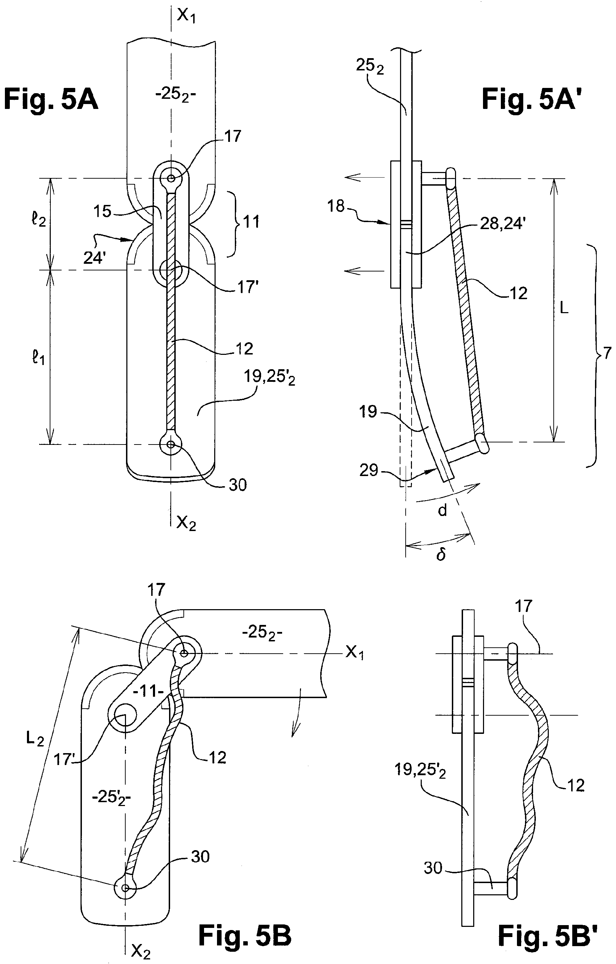

Comme représenté sur les figures 5A, 5A', 6A et 7, la longueur « L » du câble 12 est déterminée pour que celui-ci exerce une traction donnée sur les points d'attache de ses deux extrémités, quand les parties supérieure 6 et inférieure 7 sont pivotées l'une par rapport à l'autre dans le sens de tendre à l'alignement de leurs axes X₁ et X₂, c'est-à-dire quand l'articulation du genou est en position d'extension et, au contraire, comme représenté sur les figures 5'A, 5'B et 6B, soit détendu quand les parties supérieure 6 et inférieure 7 sont pivotées en sens inverse, c'est-à-dire quand l'articulation du genou est en position de flexion : à titre d'exemple, si l₂ qui est la distance entre les deux axes 17, 17' communs de la biellette 15 et l₁ la distance entre l'axe commun 17' de la biellette avec l'autre point d'attache 30 du câble 12, celui-ci peut avoir une longueur L = 147,5 mm, pour l₂ = 50 mm et l₁ = 100 mm ; la différence de 2,5 mm entre L et l₁ + l₂. crée une force de tension dans le bras 19 en position d'extension : celui-ci s'écarte alors de sa position au repos pour compenser cette différence, soit avec les valeurs ci-dessus, d'une distance « d » à son extrémité distale correspondant à un angle δ de 7° environ.

Suivant les modes de réalisation des figures 5 et 6, le bras 19 est réalisé en matériau ressort de section plus mince du côté de son extrémité proximale 28 que distale 29, et son extrémité proximale 28 est intégrée au deuxième moyen d'articulation 11 dans lequel elle est fixée et pivote autour de leur axe commun 17' avec la biellette 15 quand le genou fléchit comme représenté sur la figure 5B ; dans cette position, la longueur L₂ entre les deux points d'attache 17, 30 du câble 12 est inférieure à la longueur L de celui-ci qui est alors détendu, relâchant son effort sur l'orthèse et donc le genou.

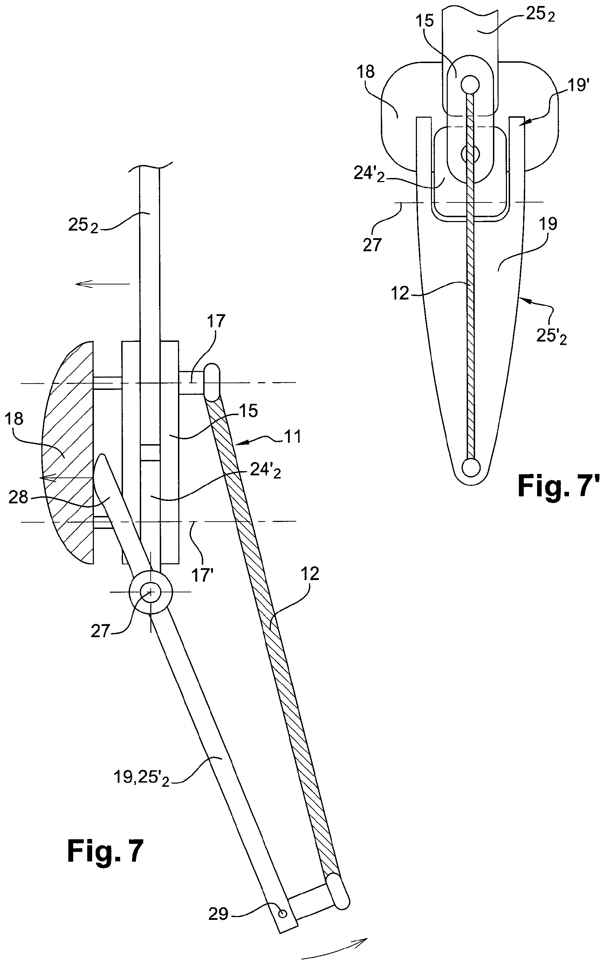

Dans un autre mode de réalisation, tel que représenté sur les figures 7 et 7' en vue de profil et en vue de côté, le bras 19 est rigide et articulé autour d'un axe 27 perpendiculaire à celui commun 17' de la biellette 15 avec l'extrémité 24'₂ de la pièce support 25'₂ : cette pièce est ainsi constituée elle-même de deux parties, celle constituant le bras 19 ayant alors son extrémité proximale 28 distincte de l'extrémité 24'₂ et pivotant avec l'ensemble du bras pour déplacer une pièce poussoir 18 qui s'avance vers le premier moyen d'articulation 10 situé de l'autre côté de l'orthèse, soit en fait contre le genou, quand les parties supérieure 6 et inférieure 7 sont pivotées dans le sens de tendre à l'alignement de leurs axes X₁, X₂, comme décrit précédemment pour d'autres modes de réalisation, et s'éloigne de ce premier moyen d'articulation quand lesdites parties supérieure et inférieure sont pivotées dans le sens d'augmenter l'angle α + β relatif entre leurs axes X₁ et X₂ : ainsi, dans ce mode de réalisation, suivant les figures 7 et 7' le câble 12 assure non seulement la fonction de force de traction sur l'orthèse et donc sur le côté du genou, comme dans le mode de réalisation des figures 5 et 6, mais également la fonction de poussée du deuxième moyen d'articulation 11 dont fait partie la pièce poussoir 18.

Quels que soient les modes de réalisation des trois dispositifs d'actions sur le genou, tels que décrits ci-dessus, la combinaison des effets de ces trois dispositifs, qui ne sont actifs qu'en position d'extension du genou, soit quand α + β varie de 0 à 35°, d'une part assure la correction de la déviation latérale initiale et, d'autre part, soulage la pression d'écrasement interosseux sur le côté du pincement du genou provoquant justement la déviation. Ces trois dispositifs relâchent leurs actions d'écartement, de traction et de poussée au-delà de 35°, soit en position de flexion, permettant au genou de reprendre une position plus confortable pour le patient.

La combinaison de ces trois effets par la complémentarité des trois dispositifs décrits précédemment et tel que représenté en exemple sur les figures jointes, assure une efficacité maximum dans le redressement de l'articulation du genou, qui soit de type Valgus ou Varus.

## Revendications

1. Orthèse de genou comprenant deux parties (6,7), la première (6) dite supérieure et la deuxième (7) dite inférieure, munies chacune d'au moins un moyen d'attache (8, 9) reliant des pièces supports (25, 25'), apte à les fixer respectivement sur une cuisse et sur une jambe d'un membre inférieur d'une personne, présentant une déviation latérale et initiale anormale (γ), et ayant une forme générale circulaire adaptable respectivement à la dite cuisse et à la dite jambe et avec le même axe respectivement (X₁) et (X₂) que celles-ci, et tel qu'ainsi elles suivent le mouvement de cette cuisse et de cette jambe, les deux dites parties (6, 7) étant reliées par des premier et deuxième moyens d'articulation (10, 11) bicentrique, destinés à être positionnés latéralement de part et d'autre du genou et adaptés à autoriser le pivotement des parties supérieure (6) et inférieure (7) autour chacune d'un axe commun (16, 16', 17, 17') entre les extrémités (24, 24') des pièces supports (25, 25') de celles-ci et une pièce intermédiaire formant biellette (14, 15) du moyen d'articulation correspondant (10, 11), et tel que le premier moyen d'articulation (10) correspond au côté de l'orthèse vers lequel s'incline la déviation initiale (γ), et le deuxième moyen d'articulation (11) correspondant au côté de l'orthèse opposée à la déviation initiale (γ) comporte une pièce poussoir (18) ayant une action de poussée sur le genou, **caractérisée en ce que** le moyen d'articulation (10), d'une part, écarte les deux axes communs (16, 16'), entre sa biellette (14) et les pièces support (25₁, 25'₁) des parties supérieure (6) et inférieure (7), quand celles-ci sont pivotées dans le sens de tendre à l'alignement de leurs axes (X₁) et (X₂) et, d'autre part, rapproche ces deux axes communs (16, 16'), quand lesdites parties supérieure et inférieure sont pivotées dans le sens d'augmenter l'angle (α + β) relatif entre leurs axes (X₁) et (X₂), et la pièce poussoir (18) du deuxième moyen d'articulation (11) s'avance vers le premier moyen d'articulation (10) situé de l'autre côté de l'orthèse, quand les parties supérieure (6) et inférieure (7) sont pivotées dans le sens de tendre à l'alignement de leurs axes (X₁) et (X₂), et s'éloigne de ce premier moyen d'articulation quand les dites parties supérieure (6) et inférieure (7) sont pivotées dans le sens d'augmenter l'angle (α + β) relatif entre leurs axes (X₁) et (X₂).

2. Orthèse de genou suivant la revendication 1,
**caractérisée en ce que** les dispositifs de poussée et d'écartement des deux moyens d'articulation (10, 11) bicentrique ne sont actifs qu'en position d'extension du genou, soit quand l'angle (α + β) varie de 0 à 35°, et relâchent leurs actions d'écartement et de poussée au-delà de 35°, soit en position de flexion.

3. Orthèse de genou suivant l'une quelconque des revendications 1 et 2, **caractérisée en ce que** la biellette (14) comporte un logement intérieur doté au moins d'une crémaillère (20) et dans lequel sont guidés et tournent deux roues dentées (21) coopérant avec cette crémaillère, lesquelles roues dentées étant fixées et solidaires chacune de l'extrémité (24₁, 24'₁) d'une pièce support (25₁, 25'₁) respectivement des parties supérieure (6) et inférieure (7) de l'orthèse.

4. Orthèse de genou suivant l'une quelconque des revendications 1 à 3, **caractérisée en ce que** la biellette (14) comporte un logement intérieur doté de deux cavités dans chacune desquelles est guidée et tourne une came fixée et solidaire chacune de l'extrémité (24₁, 24'1) d'une pièce support respectivement des parties supérieure et inférieure de l'orthèse, chaque came s'appuyant sur la forme intérieure de sa cavité en forme de rampe.

5. Orthèse de genou suivant l'une quelconque des revendications 1 à 4, **caractérisée en ce que** ladite orthèse comporte un câble (12) reliant l'axe commun (17), entre la biellette (15) du moyen d'articulation (11), correspondant au côté de l'orthèse opposée à la déviation initiale (γ), et la partie supérieure (6) de l'orthèse, avec l'extrémité distale (29) d'un bras (19) formant une des pièces supports (25') de la partie inférieure (7) de l'orthèse et disposé dans une direction quasi parallèle à l'axe (X₂) de celle-ci, la longueur du câble étant déterminée pour que celui-ci exerce une traction donnée sur les points d'attache de ses deux extrémités quand les parties supérieure (6) et inférieure (7) sont pivotées l'une par rapport à l'autre dans le sens de tendre à l'alignement de leurs axes (X₁, X₂), et au contraire soit détendu quand les parties supérieure (6) et inférieure (7) sont pivotées en sens inverse.

6. Orthèse de genou suivant l'une quelconque des revendications 1 à 5, **caractérisée en ce que** la pièce poussoir (18) forme la biellette (15) de l'articulation, laquelle biellette est de forme hélicoïdale sur au moins un quart de sa surface (22₁, 23₁) autour de ses deux axes (17, 17'), lesquelles formes (22, 23) constituent des rampes qui s'appuient et coopèrent avec des extrémités (24₂, 24'₂) des pièces supports (25₂, 25'₂) fixées respectivement aux parties supérieure et inférieure de l'orthèse et de forme également hélicoïdales (22₂, 23₂) formant également rampe, de même pas et de même axe commun (17, 17') et lesdites extrémités (24₂, 24'₂) des pièces supports comportant chacune une portion de couronne dentée (26, 26') de même axe (17, 17') et qui coopèrent l'une avec l'autre.

7. Orthèse de genou suivant la revendication 5,
**caractérisée en ce que** le bras (19) est réalisé en matériau ressort de section plus mince du côté de son extrémité proximale (28) que distale (29), et son extrémité proximale (28) est intégrée au deuxième moyen d'articulation (11) dans lequel elle est fixée et pivote autour de leur axe commun (17') avec la biellette (15).

8. Orthèse suivant la revendication 5,
**caractérisée en ce que** le bras (19) est rigide et articulé autour d'un axe (27) perpendiculaire à celui commun (17') de la biellette (15) avec l'extrémité (24'₂) de la pièce support (25'₂), celle-ci étant ainsi constituée de deux parties, celle constituant le bras (19) ayant alors son extrémité proximale (28) distincte de l'extrémité (24'₂) et pivotant avec l'ensemble du bras pour déplacer une pièce poussoir (18) qui s'avance vers le premier moyen d'articulation (10) situé de l'autre côté de l'orthèse quand les parties supérieure (6) et inférieure (7) sont pivotées dans le sens de tendre à l'alignement de leurs axes (X₁, X₂), et s'éloigne de ce premier moyen d'articulation quand les dites parties supérieure et inférieure sont pivotées dans le sens d'augmenter l'angle (α + β) relatif entre leurs axes (X₁) et (X₂).

## Patentansprüche

1. Knieorthese, die zwei Teile (6, 7) umfasst, den ersten (6), der oberer genannt wird, und den zweiten (7), der unterer genannt wird, die jeweils mit mindestens einem Befestigungsmittel (8, 9) versehen sind, das Stützteile (25, 25') verbindet, die geeignet sind, um sie jeweils auf einem Oberschenkel und auf einem Bein eines unteren Gliedmaßes einer Person zu befestigen, das eine anormale laterale und initiale Verschiebung (γ) aufweist, und die eine allgemeine Kreisform haben, die jeweils an den Oberschenkel und an das Bein anpassbar sind, und mit jeweils derselben Achse (X₁) und (X₂) wie diese, und derart, dass sie der Bewegung dieses Oberschenkels und dieses Beins folgen, wobei die zwei Teile (6, 7) durch erste und zweite Mittel zum bizentrischen Anlenken (10, 11) verbunden sind, die dazu bestimmt sind, seitlich auf jeder Seite des Knies positioniert und angepasst zu werden, um das Schwenken des oberen Teils (6) und des unteren Teils (7) jeweils um eine gemeinsame Achse (16, 16', 17, 17') zwischen den Enden (24, 24') der Stützteile (25, 25') dieser zu gestatten, und ein Zwischenteil, das einen Schwenkarm (14, 15) des entsprechenden Anlenkmittels (10, 11) bildet, und derart, dass das erste Anlenkmittel (10) der Seite der Orthese entspricht, zu der sich die initiale Verschiebung (γ) neigt, und das zweite Anlenkmittel (11), das der Seite der Orthese entspricht, die der initialen Verschiebung (γ) entgegengesetzt ist, ein Schiebeteil (18) umfasst, das eine Schubwirkung auf das Knie hat, **dadurch gekennzeichnet, dass** das Anlenkmittel (10) einerseits die zwei gemeinsamen Achsen (16, 16') zwischen seinem Schwenkarm (14) und den Stützteilen (251, 25'1) des oberen Teils (6) und des unteren Teils (7) beabstandet, wenn diese in die Richtung des Tendierens zum Ausrichten mit ihren Achsen (X₁) und (X₂) geschwenkt werden, und andererseits diese zwei gemeinsamen Achsen (16, 16') annähert, wenn der obere Teil und der untere Teil in die Richtung zum Erhöhen des entsprechenden Winkels (α + β) zwischen ihren Achsen (X₁) und (X₂) geschwenkt werden, und das Schiebeteil (18) des zweiten Anlenkmittels (11) zu dem ersten Anlenkmittel (10), das auf der anderen Seite der Orthese liegt, vorläuft, wenn der obere Teil (6) und der untere Teil (7) in die Richtung des Tendierens zum Ausrichten mit ihren Achsen (X₁) und (X₂) geschwenkt werden, und sich von diesem ersten Anlenkmittel entfernt, wenn der obere Teil (6) und der untere Teil (7) in die Richtung des Erhöhens des entsprechenden Winkels (α + β) zwischen ihren Achsen (X₁) und (X₂) geschwenkt werden.

2. Knieorthese nach Anspruch 1, **dadurch gekennzeichnet, dass** die Schub- und Beabstandungsvorrichtungen der zwei Mittel zum bizentrischen Anlenken (10, 11) nur in Streckposition des Knies aktiv sind, das heißt, wenn der Winkel (α + β) von 0 bis 35° variiert, und ihre Beabstandungs- und Schubaktion über 35° hinaus, das heißt in Biegeposition, freigeben.

3. Knieorthese nach einem der Ansprüche 1 und 2,
**dadurch gekennzeichnet, dass** der Schwenkarm (14) eine Innenaufnahme umfasst, die mit mindestens einer Zahnstange (20) versehen ist, und in der zwei Zahnräder (21) geführt werden und sich drehen, die mit dieser Zahnstange zusammenwirken, wobei die Zahnräder befestigt und jeweils mit dem Ende (24₁, 24'₁) eines Stützteils (25₁, 25'₁) jeweils des oberen Teils (6) und des unteren Teils (7) der Orthese fest verbunden sind.

4. Knieorthese nach einem der Ansprüche 1 bis 3,
**dadurch gekennzeichnet, dass** der Schwenkarm (14) eine Innenaufnahme umfasst, die mit zwei Hohlräumen versehen ist, wobei in jedem ein Nocken geführt wird und sich dreht, der befestigt und jeweils mit dem Ende (24₁, 24'₁) eines Stützteils jeweils des oberen Teils und des unteren Teils der Orthese fest verbunden ist, wobei sich jeder Nocken auf die Innenform seines Hohlraums in Rampenform stützt.

5. Knieorthese nach einem der Ansprüche 1 bis 4,
**dadurch gekennzeichnet, dass** die Orthese ein Kabel (12) umfasst, das die gemeinsame Achse (17) zwischen dem Schwenkarm (15) des Anlenkmittels (11), das der Seite der Orthese, die der inititalen Verschiebung (γ) entspricht, entgegengesetzt ist, und dem oberen Teil (6) der Orthese mit dem distalen Ende (29) eines Arms (19) verbindet, der eines der Stützteile (25') des unteren Teils (7) der Orthese bildet und in eine Richtung so gut wie parallel zu der Achse (X₂) dieser angeordnet ist, wobei die Länge des Kabels festgelegt ist, damit dieses einen gegebenen Zug auf die Befestigungsstellen seiner zwei Enden ausübt, wenn der obere Teil (6) und der untere Teil (7) zueinander in die Richtung des Tendierens zum Ausrichten mit ihren Achsen (X₁, X₂) geschwenkt werden, und im Gegensatz entspannt wird, wenn der obere Teil (6) und der untere Teil (7) in umgekehrte Richtung geschwenkt werden.

6. Knieorthese nach einem der Ansprüche 1 bis 5,
**dadurch gekennzeichnet, dass** das Schiebeteil (18) den Schwenkarm (15) der Anlenkung bildet, wobei der Schwenkarm auf mindestens einem Viertel seiner Oberfläche (22₁, 23₁) um diese zwei Achsen (17, 17') Schraubenformen hat, wobei diese Formen (22, 23) Rampen bilden, die sich auf die Enden (24₂, 24'₂) der Stützteile (25₂, 25'₂), die jeweils an dem oberen Teil und dem unteren Teil der Orthese befestigt sind und ebenfalls Schraubenformen (22₂, 23₂) haben, die ebenfalls Rampen bilden, stützen und mit ihnen zusammenwirken, mit derselben Steigung und derselben gemeinsamen Achse (17, 17'), und die Enden (24₂, 24'₂) der Stützteile jeweils einen Zahnradkranzabschnitt (26, 26') mit derselben Achse (17, 17') umfassen und die miteinander zusammenwirken.

7. Knieorthese nach Anspruch 5, **dadurch gekennzeichnet, dass** der Arm (19) aus Federwerkstoff mit einem Querschnitt hergestellt ist, der auf der Seite seines proximalen Endes (28) dünner ist als auf der seines distalen Endes (29), und sein proximales Ende (28) in das zweite Anlenkmittel (11) integriert ist, in dem es um ihre gemeinsame Achse (17') mit dem Schwenkarm (15) befestigt ist und sich dreht.

8. Orthese nach Anspruch 5, **dadurch gekennzeichnet,**
**dass** der Arm (19) starr ist und um eine Achse (27) senkrecht zu der gemeinsamen Achse (17') des Schwenkarms (15) mit dem Ende (24'₂) des Stützteils (25'₂) angelenkt ist, wobei dieses daher aus zwei Teilen besteht, dem, das den Arm (19) bildet, der daher sein proximales Ende (28) von dem Ende (24'₂) getrennt hat und mit der Einheit des Arms schwenkt, um ein Schiebeteil (18) zu verschieben, das zu dem ersten Anlenkmittel (10) vorläuft, das auf der anderen Seite der Orthese liegt, wenn der obere Teil (6) und der untere Teil (7) in die Richtung zum Tendieren zum Ausrichten mit ihren Achsen (X₁, X₂) geschwenkt werden, und sich von diesem ersten Anlenkmittel entfernt, wenn der obere Teil und der untere Teil in die Richtung zum Erhöhen des entsprechenden Winkels (α + β) zwischen ihren Achsen (X₁) und (X₂) geschwenkt werden.

## Claims

1. Knee orthosis comprising two parts (6, 7), the first (6) being the upper part and the second (7) being the lower part, said parts each being provided with at least one attachment means (8, 9) which links support components (25, 25') and is suitable for fixing said parts respectively to a thigh and to a leg of a lower limb of a person presenting an abnormal initial lateral deviation (γ), and with a circular general shape that is adaptable respectively to said thigh and to said leg and with the same axis respectively (X₁) and (X₂) as those, and such that they thus follow the movement of this thigh and of this leg, said two parts (6, 7) being connected via first and second bi-centric articulation means (10, 11) which are intended to be positioned laterally on each side of the knee and are designed to allow the upper part (6) and lower part (7) to each pivot about a common axis (16, 16', 17, 17') between the ends (24, 24') of the support components (25, 25') thereof and an intermediate component forming a connection rod (14, 15) of the corresponding articulation means (10, 11), and such that the first articulation means (10) corresponds to the side of the orthosis toward which the initial deviation (γ) inclines, and the second articulation means (11), corresponding to the side of the orthosis opposite the initial deviation (γ), has a pusher component (18) having a pushing action on the knee, charaterized in that the articulation means (10), on the one hand, moves apart the two common axes (16, 16'), between its connection rod (14) and the support components (25₁, 25'₁) of the upper part (6) and lower part (7), when these are pivoted in the sense of tending toward the alignment of their axes (X₁) and (X₂), and, on the other hand, brings these two common axes (16, 16') closer when said upper and lower parts are pivoted in the sense of increasing the relative angle (α + β) between their axes (X₁) and (X₂), and the pusher component (18) of the second articulation means (11) advances toward the first articulation means (10), situated on the other side of the orthosis, when the upper part (6) and the lower part (7) are pivoted in the sense of tending toward the alignment of their axes (X₁) and (X₂), and which moves away from this first articulation means when the said upper part (6) and the said lower part (7) are pivoted in the sense of increasing the relative angle (α + β) between their axes (X₁) and (X₂).

2. Knee orthosis according to Claim 1, **characterized**
**in that** the pushing and spacing-apart devices of the two bi-centric articulation means (10, 11) are only active in the extension position of the knee, i.e. when the angle (α + β) varies from 0 to 35°, and they relax their spacing-apart and pushing actions beyond 35°, i.e. in the position of flexion.

3. Knee orthosis according to either of Claims 1 and
2, **characterized in that** the connection rod (14) has an internal seat which is equipped at least with a rack (20) and in which two toothed wheels (21) cooperating with this rack are guided and turn, said toothed wheels being fixed and each rigidly connected to the end (24₁, 24'₁) of a support component (25₁, 25'₁) respectively of the upper part (6) and lower part (7) of the orthosis.

4. Knee orthosis according to any one of Claims 1 to
3, **characterized in that** the connection rod (14) has an internal seat equipped with two cavities, in each of which is guided and turns a cam which is fixed and rigidly connected in each case to the end (24₁, 24'₁) of a support component respectively of the upper and lower parts of the orthosis, each cam bearing on the internal shape of its ramp-shaped cavity.

5. Knee orthosis according to any one of Claims 1 to
4, **characterized in that** said orthosis has a cable (12) linking the common axis (17), between the connection rod (15) of the articulation means (11), corresponding to the side of the orthosis opposite the initial deviation (γ), and the upper part (6) of the orthosis, to the distal end (29) of an arm (19) forming one of the support components (25') of the lower part (7) of the orthosis and arranged in a direction almost parallel to the axis (X₂) thereof, the length of the cable being determined such that it exerts a given traction on the attachment points of its two ends when the upper part (6) and lower part (7) are pivoted in relation to each other in the sense of tending toward the alignment of their axes (X₁, X₂) and, by contrast, is untensioned when the upper part (6) and lower part (7) are pivoted in the opposite sense.

6. Knee orthosis according to any one of Claims 1 to
5, **characterized in that** the pusher component (18) forms the connection rod (15) of the articulation, which connection rod is of helicoidal shapes over at least a quarter of its surface (22₁, 23₁) around its two axes (17, 17'), which shapes (22, 23) constitute ramps which bear on and cooperate with ends (24₂, 24'₂) of the support components (25₂, 25'₂) fixed respectively to the upper and lower parts of the orthosis and having likewise helicoidal shapes (22₂, 23₂) likewise forming ramps of a same pitch and with the same common axis (17, 17'), and said ends (24₂, 24'₂) of the support components each having a portion of a toothed ring (26, 26') with the same axis (17, 17'), which cooperate with each other.

7. Knee orthosis according to Claim 5, **characterized**
**in that** the arm (19) is made of resilient material with a thinner cross section at its proximal end (28) than at its distal end (29), and its proximal end (28) is integrated in the second articulation means (11), in which it is fixed and pivots around their common axis (17') with the connection rod (15).

8. Orthosis according to Claim 5, **characterized in**
**that** the arm (19) is rigid and articulated about an axis (27) perpendicular to the common axis (17') of the connection rod (15) with the end (24'₂) of the support component (25'₂), the latter thus being composed of two parts, the part constituting the arm (19) then having its proximal end (28) distinct from the end (24'₂) and pivoting with the whole of the arm in order to move a pusher component (18) which advances toward the first articulation means (10) situated on the other side of the orthosis when the upper part (6) and the lower part (7) are pivoted in the sense of tending toward the alignment of their axes (X₁, X₂), and which moves away from this first articulation means when the said upper and the said lower parts are pivoted in the sense of increasing the relative angle (α + β) between their axes (X₁) and (X₂).
